# EUROPÄISCHE PATENTSCHRIFT

(11) **EP 1 161 557 B1**
(45) Veröffentlichungstag und Bekanntmachung des Hinweises auf die Patenterteilung: **17.09.2003**
(21) Anmeldenummer: 00925110.9
(22) Anmeldetag: 13.03.2000
(51) Int. Cl.: C12Q 1/37, G01N 33/574

(54) **CHEMOSENSITIVITÄTSMESSUNG ÜBER CASPASE-AKTIVITÄT**
DETERMINATION OF THE CHEMOSENSITIVITY VIA THE CASPASE ACTIVITY
MESURE DE LA CHIMIOSENSIBILITE PAR L'INTERMEDIAIRE DE L'ACTIVITE CASPASE

(30) Priorität: 12.03.1999 DE 19910956; 30.04.1999 EP 99108495
(43) Veröffentlichungstag der Anmeldung: 12.12.2001
(73) Patentinhaber: Evotec Technologies GmbH, 40225 Düsseldorf (DE)
(72) Erfinder: MEYER-ALMES, Franz, Josef, D-58636 Iserlohn (DE)
(74) Vertreter: Meyers, Hans-Wilhelm, Dr.Dipl.-Chem.
(86) Internationale Anmeldenummer: EP0002174
(87) Internationale Veröffentlichungsnummer: WO00054049

(56) Entgegenhaltungen:
- WO-A-98/13517
- WO-A-99/09208
- WO-A-99/18856
- WO-A-99/24620
- FULDA, S. ET AL.: "Molecualr determinants of apoptosis induced by cytotoxic drugs" KLINISCHE PÄDIATRIE, Bd. 210, Nr. 4, Juli 1998 (1998-07), Seiten 148-152, XP000900914
- FULDA ET AL: "The CD95 (APO-1/Fas) system mediates drug-induced apoptosis in neuroblastoma cells" CANCER RESEARCH,US,AMERICAN ASSOCIATION FOR CANCER RESEARCH, BALTIMORE, MD, Bd. 57, Nr. 17, 1. September 1997 (1997-09-01), Seiten 3823-3829, XP002118945 ISSN: 0008-5472
- COHEN G: "Caspase: the executioners of apoptosis" BIOCHEMICAL JOURNAL,GB,PORTLAND PRESS, LONDON, Bd. 326, 15. August 1997 (1997-08-15), Seiten 1-16, XP002091927 ISSN: 0264-6021 in der Anmeldung erwähnt
- STENNICKE, H.R. AND SALVESENS, G.S.: "Biochemical characteristics of caspases-3, -6, -7 and -8" THE JOURNAL OF BIOLOGICAL CHEMISTRY, Bd. 272, Nr. 41, 1997, Seiten 25719-25723, XP002146144 in der Anmeldung erwähnt

## Beschreibung

Die Erfindung betrifft ein Verfahren zur Bestimmung der Chemosensitivität von Zellen gegenüber mindestens einer Substanz durch Messung der durch die mindestens eine Substanz ausgelösten Apoptose.

### Chemosensitivitätstests

Die Behandlungs- und Heilungserfolge von Tumorerkrankungen haben seit der Einführung der Chemotherapie stark zugenommen. Beispielsweise lag die Überlebensrate bei kindlicher akuter lymphatischer Leukämie (ALL) Mitte der 60er Jahre bei weniger als 10%. Heutzutage liegt die Heilungschance bei über 70%. Die Medikamente, sog. Zytostatika, werden nach bestimmten Therapieplänen allein oder in Kombination mit anderen Wirkstoffen verabreicht. Mittlerweile gibt es eine unüberschaubare Anzahl verschiedener Therapiepläne, die empirisch ermittelt und ständig weiterentwickelt werden. Das Hauptkriterium für einen verbesserten Therapieplan ist eine bessere Überlebensrate (clinical outcome). Dieses Kriterium trifft für die Mehrheit der Patienten zu. Jedes Individuum besitzt aber eine unterschiedliche Ausstattung an Zellen mit unterschiedlichen Eigenschaften. Dies trifft insbesondere auf die Eigenschaften von Tumorzellen zu. In einigen Studien konnte gezeigt werden, dass die Therapie für eine Subpopulation extrem gut wirkt, während sie für andere Patienten aufgrund von medikamentresistenten Tumoren wenig oder überhaupt nicht wirksam ist. (Lacombe et al., Blood, 84, 716-723 (1994), Smit et al. International Journal of Cancer 51, 72-78 (1992)). Dabei konnte gezeigt werden, dass nicht nur die Wirksamkeit verschiedener Medikamente, sondern auch die wirksame Dosis eines Medikamentes individuell unterschiedlich sein kann. Die Ermittlung der individuellen Dosierung eines Medikamentes ist für den Patienten von Bedeutung, damit er einerseits soviel an Medikamenten bekommt, wie für seine Heilung notwendig ist, und damit andererseits die toxischen Auswirkungen der Therapie und die Wahrscheinlichkeit einer durch die Chemotherapie induzierten sekundären Krebserkrankung so gering wie möglich gehalten werden. So gut die aktuell gebräuchlichen Therapiepläne auch sind, die Fortschritte der letzten Jahre stagnieren hinsichtlich der mittleren Überlebensrate. Eine weitere deutliche Verbesserung der Chemotherapie sollte durch die Erstellung individuell angepasster Therapiepläne möglich sein.

Um diese Problematik anzugehen haben einige Forscher versucht, die individuelle Sensitivität von Patiententumoren gegenüber Zytostatika *in vitro* zu messen. Die meisten Chemosensitivitätstests, die gegenwärtig verwendet werden, basieren wenigstens teilweise auf dem von Salmon (Salmon et al. Science, 197, 461-463 (1977)) entwickelten Agar-Tumorkultur-Test. Diese Tests messen die Zellproliferation.

Ein zweiter Typ von Chemosensitivitätstests umfasst den Ausschluss von (Fluoreszenz)-Farbstoffen oder die Freisetzung des radioaktiven Chromisotops ⁵¹Cr, mit dem die Zerstörung der Zellmembran durch direkte oder indirekte Zytostatikawirkung gemessen wird. Eine Modifikation des früheren Farbstoffausschluss-Tests ist der sogenannte DiSC-Assay (Weisenthal, Kern Oncology 5: 92-103 (1991)), der durch einen zweiten Färbevorgang zusätzlich zwischen normalen und Tumorzellen differenziert.

Der dritte Typ von Chemosensitivitätstests bestimmt Parameter des zellulären Metabolismus als Maß für die Schädigung durch Zytostatika. Dieser Testtyp umfasst den radiometrischen BACTEC-Test (von Hoff D., Forseth B., Warfel L., in Salmon Trent(Eds.) Human tumor cloning, pp. 656-657, Grune & Stratton, Orlando (1984)) , den MTT-Test (Freund A. et al. European Journal of Cancer 34:895-901 (1998), Kaspers G.J.L. Blood 92:259-266 (1998), Pieters R. et al. Leukemia 12, 1344-1348 (1998), Klumper E. et al. British Journal of Haematology 93:903-910 (1996), Hwang W.S. et al. Leukemia Research 17:685-688 (1993)) und seinen Variationen, den ATP-Test (Kangas L., Gronroos M., Nieminen A., Medical Biology 62: 338-343 (1984)) und den sogenannten FCA-Test (Meitner P., Oncology 5: 75-81, (1991), Rotman B, Teplitz C., Dickinson K., Cozzolino J., Cellular and Developmental Biology 24: f1137-1146 (1988)).

Agar-Kultur-Assays haben den großen Nachteil, dass bei weitem nicht alle Tumorzellen in den Agar-Kulturen wachsen. Dies gilt insbesondere für lymphatische Leukämien und Lymphome (Veerman A.J.P., Pieters R. British Journal of Haematology 74:381-384 (1990)). Beim bekanntesten Vertreter dieses Assay-Typs, dem Clonogenen Assay, liegt der Prozentsatz von auswertbaren Zellpopulationen nur bei 30-40%. Die Zellen müssen sehr lange (10-20 Tage) wachsen, bevor die Auswertung erfolgt. Außerdem ist der Arbeitsaufwand immens.

Die Farbstoff-Ausschluss-Tests wie auch der ⁵¹Cr-Freisetzungstest bestimmen den Anteil von Zellen mit defekter Zellmembran. Diese Tests beinhalten häufig eine Fixierung und anschließende mikroskopische Auswertung der Zellen bzw. die Messung der Radioaktivität im Überstand. Solche Tests sind aufgrund der Messung eines recht groben Parameters, der Zellmembranintegrität, unspezifisch und können nicht zwischen einer spezifischen Anti-Tumor-Wirkung einer Substanz oder einer physikalischen oder z.B. durch eine starke Verätzung oder Oxidation verursachten Zellschädigung durch eine Substanz unterscheiden. Tests dieses Typs sind folglich auch bei Substanzen positiv, die generell alle Zellen schädigen und damit nicht als Antitumor-Medikament geeignet sind. Der wohl aussagekräftigste Test des Farbstoff-Ausschluss-Typs ist der DiSC-Test. Durch zweifache Anfärbung kann er im Gegensatz zu anderen Farbstoff-Ausschlusstests zwischen der Zellschädigung von Tumorzellen und normalen Zellen unterscheiden. Die Zweifach-Färbung und die anschließende mikroskopische Auswertung sind aber extrem zeitaufwendig. Außerdem variieren die Ergebnisse abhängig von der Person, die die Auswertung durchführt, und dem Bildausschnitt, der unter dem Mikroskop untersucht wird.

Für radiometrische Tests wie z.B. den ⁵¹Cr-Freisetzungstest gelten ganz generell: Der Einsatz von radioaktiven Isotopen in diagnostischen Tests wird heutzutage wegen des Gefährdungspotentials und der Entsorgungsproblematik zunehmend vermieden. Hinzu kommt, dass inzwischen Fluoreszenz- und Luminiszenztechniken gleiche Sensitivitäten erreichen können bei häufig kürzerer Gesamttestdauer.

Von den Chemosensitivitäts-Tests, die den Metabolismus einer Zelle als Maß für die Proliferation verwenden, wird am häufigsten der MTT-Test und seine Variationen verwendet. Der MTT-Test hat nach einer 4-tägigen Inkubation der Zellen mit diversen Zytostatika eine relativ kurze Testdauer von etwa 4 Stunden. Außerdem können 96 Proben parallel in einer Mikrotiterplatte mittels eines ELISA-Auslesegerätes ausgewertet werden. Dadurch wird ein passabler Durchsatz bei relativ geringem Personalaufwand gewährleistet. Der MTT-Test basiert auf der Umsetzung von 3-[4,5-Dimethylthiazol-2-yl]-2,5-diphenyl-tetrazoliumbromid in ein blaues Formazan-Produkt. Die Umsetzung wird durch Dehydrogenasen katalysiert, die nur in lebenden Zellen aktiv sind. Der MTT-Test ist abhängig von einer konstitutiven Basisaktivität der untersuchten Zellen. Es hat sich aber herausgestellt, dass einige Zelltypen, z.B. besonders häufig einige FAB-Subtypen von akuter myeloischer Leukämie, eine stark verringerte Dehydrogenaseaktivität besitzen (Santini V. et al. Hematological Oncology 7:287-293 (1989)). Die Chemosensitivität dieser Tumorzellen kann daher nicht mit einem MTT-Test bewertet werden. Ein weiteres Problem sind die entstehenden wasserunlöslichen Formazankristalle. Diese müssen durch Zusatz von organischen Lösungsmitteln z.B. Dimethylsulfoxid oder Isopropanol in Lösung gebracht werden. Die Erfahrung hat gezeigt, dass dadurch Probleme bei der Reproduzierbarkeit des Tests auftreten.

### Apoptose

Durch Arbeiten diverser Forschergruppen (z.B. Sen, S. et al. FEBS Lett. 307:122-127, Darzynkiewicz et al. Cytometry 13: 795-808 (1992), Fulda S., Los M., Friesen C., Debatin K.M. Int. J. Cancer 76:105-114 (1998)) erscheint es wahrscheinlich, dass durch Zytostatika verursachter Zelltod generell über den Mechanismus der Apoptose verläuft. Die Apoptose stellt einen von der Zelle selbst programmierten Zelltod dar, der durch physiologische Faktoren im Organismus induziert werden kann oder durch Chemikalien wie Zytostatika ausgelöst werden kann. Der programmierte Zelltod spielt eine außerordentlich große Rolle bei der Zytostase z.B. des Immunsystems. So werden beispielsweise T-Zellen, die gegen körpereigene Strukturen gerichtet sind, durch die Apoptose aus dem Organismus entfernt. Ansonsten kann es zu Symptomen einer Autoimmunerkrankung kommen, z.B. Lupus Erythematodes, arthritische Erkrankungen oder der Erkrankung durch einen T-Zell Tumor. Substanzen wie der von Makrophagen synthetisierte Tumornekrosefaktor alpha sind natürliche Induktoren der Apoptose.

Apoptose verläuft nach einem programmierten, einheitlichen Schema, bei dem u.a. bestimmte cysteinhaltige Proteasen (Caspasen) aktiviert werden (Cohen G.M. Biochem. J. 326:1-16 (1997)). Die Aktivierung von Caspasen geschieht ausschließlich durch Apoptose und ist daher ein spezifischer Parameter für die Quantifizierung von Apoptose. Bis zu diesem Zeitpunkt sind die Zellmembranen noch intakt und undurchlässig für DNA-bindende Fluoreszenzfarbstoffe wie z.B. Propidiumiodid. Daher ist die Caspaseaktivität ein Parameter für die Wirkung von Zytostatika, der deutlich früher auftritt, als die Stunden später erfolgende Auflösung der Zellmembran.

Neben dem Zelltod durch Apoptose existiert eine andere Form von Zelltod, die z.B. physikalisch, durch osmotischen Schock, Verätzung oder Oxidation ausgelöste Nekrose. Diese äußert sich durch stark degenerative Schäden an der Zelle.

Medenica (US 5736129) bestimmt das Ausmaß der Zellschädigung durch Zytostatika, indem er die Zellen mit Propidiumlodid anfärbt und anschließend die angefärbten Zellen im FACS (fluorescence activated cell sorter) auszählt. Dieser Ansatz hat aber den großen Nachteil, nicht genau zwischen Apoptose und Nekrose zu unterscheiden, und kann demzufolge den spezifischen Wirkmechanismus von Zytostatika nicht erfassen. Außerdem ist der Zellbedarf für eine aussagekräftige Analyse sehr hoch.

Caspase-Aktivititätstests sind zur spezifischen Detektion von Apoptose bestens geeignet, weil nekrotische Schädigungen, die für zytostatische Wirkungen nicht aussagekräftig sind, nicht erfaßt werden (siehe Tabelle 1). Die in den letzten Jahren verwandten Methoden zur Caspaseaktivitätsmessung umfassen die gelelektrophoretische Auftrennung von spezifischen Proteinsubstraten, sowie chromogene und fluorogene Tests, bei denen durch die Caspasen farbige Produkte gebildet werden (s. z.B. Cohen G.M. Biochem. J. 326:1-16 (1997), Stennicke H.R., Salvesen G.S. J. Biol. Chem. 41:25719-25723 (1997)). Diese Tests sind alle dadurch gekennzeichnet, daß die Zellen zu einem bestimmten Zeitpunkt nach Induktion zunächst sedimentiert und mit Puffer gewaschen werden und anschließend durch einen Lysepuffer zerstört werden, damit die Caspasen, die sich bis dahin im Zellinnern befunden haben, das zugegebene Substrat umsetzen können.

So beschreibt ein Katalog der Firma Clontech, Palo Alto, California, Katalog 98/99, Seiten 71 - 72 (1998) ein Verfahren zur Bestimmung der Apoptose von Zellen als Antwort auf die Einwirkung von Substanzen. Auch in dieser Versuchsanordnung werden gemäß den einschlägigen Arbeitsvorschriften Waschschritte durchgeführt.

WO-A-99/18856 betrifft ein Verfahren zur Chemosensitivitätsmessung von Zellen gegen Substanzen, wobei zellgängige fluorogene Substrate eingesetzt werden. Nachteilig daran ist, dass nur spezielle Substrate eingesetzt werden können.

Fulda, S. et.al. (1998, "Molecular determinants of apoptosis induced by cytotoxic drugs", Klinische Pädiatrie, Vol. 210, #4, p148-152) offenbart eine Methode zur Messung von Apoptose induzierendem Doxorubizin, Cisplatin und VP-16 mittels Durchflusscytometrie. Damit sind keine Zelllysate messbar. Außerdem ist die Durchflusscytometrie eine vergleichsweise aufwendige Methode.

WO-A-98/13517 betrifft eine Methode zur Abschätzung des Ausmaßes der Apoptose in Zellen. Dabei werden die Zellen mit einem Substrat für Proteasen der ICE/Ced-3-Familie beladen und die Umsetzung des Substrates durch die Protease mittels Durchflusscytometrie verfolgt. Damit sind jedoch keine Lysate messbar. Wie angeführt, ist die Durchflusscytometrie verhältnismäßig aufwendig. Die Beladung der Zellen setzt darüber hinaus eine Permeabilisierung der Zellen voraus. Dies kann zu Komplikationen führen. Die Zellen sind durch die Permeabilisierungsbehandiung oft nicht hinreichend authentisch, sondern entsprechen eher einem artifiziellen System.

Aus dem Waschvorgang ergibt sich aber hinsichtlich einer einfachen, schnellen Testdurchführung mit geringem Zellmaterialbedarf folgendes Problem:

Die Apoptose und damit die Entwicklung der Caspaseaktivität ist ein dynamischer Prozess, bei dem die Zelle nach einer gewissen Zeit in die sogenannte sekundäre Nekrose übergeht. Beim Waschvorgang werden daher die Caspasen dieser zerstörten Zellen entfernt.

Dadurch erhält man ein Signal für aktuell in Apoptose befindliche Zellen, deren Zahl irgendwann durch sekundäre Nekrose abnimmt. Es ist bekannt, dass das Maximum der Zahl apoptotischer Zellen sowohl vom untersuchten Zytostatikum, als auch vom Zelltyp abhängt. A priori ist nicht bekannt, zu welchem Zeitpunkt die Caspase-Aktivität und damit das Ausmaß der Apoptose gemessen werden muss. Daraus folgt, dass im Prinzip für jeden Patienten mehrere Proben mit z.B. Tumorzellen unterschiedlichen Inkubationszeiten hinsichtlich der Chemosensitivität bestimmt werden müssen. Der Bedarf an primären Patientenzellen wäre dann derartig hoch, dass nur noch die Chemosensitivität eines oder weniger Zytostatika getestet werden könnte. Außerdem wäre der Zeitund Personalaufwand für die Chemosensitivitätstestung eines einzelnen Patienten extrem hoch.

Aufgabe der vorliegenden Erfindung ist es, die genannten Nachteile des Standes der Technik zu überwinden.

Gelöst wird die Aufgabe durch ein Verfahren zur Bestimmung der Chemosensitivität von Zellen gegen mindestens eine Substanz durch Messung der durch die mindestens eine Substanz ausgelösten Apoptose, wobei die Apoptose durch kumulierte Caspase-Aktivität einer Probe mit Zellen und einem Medium bestimmt wird, indem die Probe mit der mindestens einen Substanz versetzt und inkubiert wird und nach Zerstörung der Zellen ohne vorherige Abtrennung der Zellen die kumulierte Caspase-Aktivität in der Probe gemessen wird. Das erfindungsgemäße Verfahren erlaubt insbesondere die Verwendung von schwer- oder nichtzellgängigen Substraten.

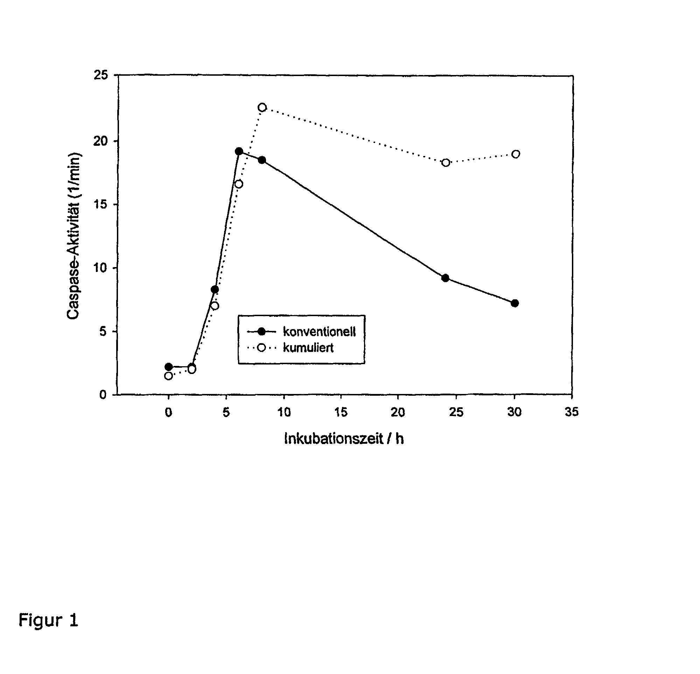

Wesentlich ist die Messung der kumulierten Caspaseaktivität. Bei diesem Ansatz werden Zellen, z.B. Tumorzellen eines Patienten, mit mindestens einer Substanz (z.B. Zytostatika) einen längeren Zeitraum inkubiert, so dass alle, auch die langsam wirkenden, wirksamen Pharmaka Zeit genug hatten, Apoptose auszulösen. Anschließend wird beispielsweise ein in einem Lysepuffer befindliches Substrat zugegeben und nach erfolgter Zell-Lyse die gesamte entstandene - kumulierte - Caspaseaktivität der Zellsuspension gemessen. Es wurde gefunden, dass durch Zytostatika aktivierte Caspasen auch nach sekundärer Nekrose apoptotischer Zellen aktiv bleiben, die kumulierte Caspaseaktität mit der Inkubationszeit steigt und ab dem Zeitpunkt, wo zunehmend sekundäre Nekrose stattfindet, auf einem hohen Level bleibt. Die herkömmlich (mit Waschschritt) bestimmte Caspaseaktivität steigt im Gleichschritt mit der kumulierten Caspaseaktivität, nimmt aber mit beginnender sekundärer Nekrose ab (siehe Figur 1). Das Prinzip der Messung der kumulierten Caspaseaktivität ermöglicht es somit, durch eine einzige Caspaseaktivitätsmessung die Wirkung von Substanzen auf die Zellen zu bestimmen . Dieser Test hat einen geringen Zellbedarf und kann in kurzer Zeit als Routinetest von Laboranten oder technischen Mitarbeitern durchgeführt werden. Durch die leichte Automatisierbarkeit ist der Personalaufwand für die Durchführung des Tests gering. Da der Test auf überall vorhandenen ELISA-Auslesegeräten durchgeführt werden kann, sind keine Geräteinvestitionen erforderlich. Um den Materialverbrauch weiter zu senken, ist eine Miniaturisierung möglich. Es konnte gezeigt werden, dass Zellen in weniger als 10 µl Medium einige Tage in Kultur gehalten werden können. Durch die Senkung der Zellzahl pro Probenkammer auf 100 bis 1000 pro Test werden hochparallele Analysen von Proben mit äußerst wenig Patientenzellen, wie z.B. aus Feinnadelbiopsien, ermöglicht. Der geringe Zellbedarf ermöglicht auch die Hochdurchsatztestung (High Throughput Screening /HTS) von unbekannten Substanzen auf anti-Tumorwirkung.

Wenn die Chemosensittvität von Zellen pathologischer Gewebe über Caspaseaktivität getestet wird, ist es von Vorteil, als Referenz gesunde Zellen des Gewebes sowie dieselben Zellen ohne Substanzzusatz mitzutesten. Insbesondere ist es sinnvoll als Kontrolle eine permanente Zell-Linie, deren Chemosensitivität bekannt ist, mitzutesten, um die Funktionalität des Tests zu dokumentieren und Fehler bei der Testdurchführung zu erkennen.

Als Substanzen kommen z.B. folgende Substanzen in Frage:
A) Zytostatika
   Abrin, Amethopterin, Acivin, Aclacinomycin A, Alanin-Mustard, Altretamin, Aminoglutethimid, Aminopterin, Amsacrin (mAMSA), div. anabolische Steroide, Anthrapyrazol, L-Asparaginase, 5-Axacytidin, Bazillus Calmette-Guerin, Bisantren, Buserelin, Busulfan, Butyryloxyethylglyoxal-dithiosemicarbazon, Camptothecin, Carbamatester, Carzinophyllin, CCNU, Chlorambucil, Chlorethylmethylcyclohexylnitrosoharnstoff, Chlorethylcyclohexylnitrosoharnstoff, Chlorodeoxyadenosin, Corticotropin, Cyproteronacetat, Chlorotrianisen, Chlorozotozin, Chromomycin-A, Cytosinarabinosid (Ara-C), BCNU, Bleomycin, Cis-Platin, Carbo-Platin, Cladribine, Cyclophosphamid, Dactinomycin, Daunomycin, Daunorubicin, Decarbacin, Doxorubicin, DTIC, Dehydroemitin, 4-Demethoxydaunorubicin, Demothydoxorubicin, Deoxydoxorubicin, Dexamethason, Dibromodulcitol, Dichloromethotrexat, Diethylstilbestrol, Bis-(2-chloropropyl)- DL-Serin, Doxifluridin, Elliptiniumacetat, 4'-Epidoxorubicin, Epirubicin, Epoetin alpha, Erythropoeitin, Esorubicin, Estradiol, Etoposid, Fluoxymesteron, Flutamid, Folsäure, Fotemustin, Ftorafur, 4-FU, Fludarabine-Phosphat, 5-FU, Floxuridin, Galactitol, Galliumnitrat, Goserelin, G-CSF, GM-CSF, Hydrea, Hexamethylmelamin (HMM), Hydrocortison, Hydroxyprogesteron, 4-Hydroperoxycyclophosphamid, ICRf 159, Idamycin, Ifosfamid, Immunglobulin IGIV, Interferon, Kobalt-Proporphyrinkomplex, Leucovorin Calcium, Leuprolid, Levadopa, Levothyroxin, Lindan, Liothyronin, Liotrix, Lomustin, Levamisole, Masoprocol, Maytansin, Menogaril, 6-Mercaptopurin, Methosalen, Methylesteron, Methyl-lomustin, Mithracin, Mithramycin, Mitotan, Mitoxantron, Methotrexat (MTX), 6 MP, Mechlorethamin-Hydrochlorid, Medroxyprogesteron, Megestrolacetat, Melphalan, Mesna, Mitomycin-C, Nandrolon, Natriumphosphat P32, Navelbin, Neocarzinostatin, Nitrofururazon, nHuIFNa, nHuIFNb, nHuIFNp, Octreotidacetat, Ondansetrone Hydrochlorid, Dinatrium-Pamidronat, Pentamethylmelamin (PMM), Pentostatin, Peptiochemio, Plicamycin, Prednimustin, Probroman, Procarbazin, Profiromycin, Paraplatin, Prednisolon, Prednison, RazoxanrIFNa-2a, Rubidazon, rIFNa-2b, rIFNb-1b, rIFNt-1b, rIL-2, rTNF, Semustin, SPG 827 (Podophyllinderivat), Spirogermanium, Streptonigrin, Somatostatin, Streptozocin, Tamoxifen, Taxol, Thio-TEPA, 6-Thioguanin, Tenoposid, Testolacton, Testosteron, 3-TGDR, rTNF, Thyroglobulin, Thyrotropin, Trilostan, Uracil-Mustard, VP-16, Vincristin (VCR), Vinblastin (VBL), Verapamil, Vindesin, Vinzelidin, Vitamin A-Säure, Vitamin A-Analoga, Zinostatin.
B) Peptide und Peptoide
C) Nukleinsäuren und -Derivate
D) Peptidnukleinsäuren (PNA's)
E) Hybride aus RNA's, DNA's, PNA's u. Derivate

Die Caspase-Aktivität kann sowohl durch Substratumsatz von fluorogenen oder chromogenen Substraten oder durch Bindung von spezifischen Markern z.B. Antikörper, F_{ab}-Fragmente, single chain Antikörper, Aptamere (Strukturen bindende Nukleinsäuren) und/oder sonstige Proteine mit Bindungsstellen für entweder unveränderte (Edukte) oder umgesetzte Caspase-Substrate (Produkte) gemessen werden. Insbesondere ist die Verwendung von Aminocoumarin-DEVD als fluorogenes Substrat geeignet.

Markermoleküle, die spezifisch entweder Edukte oder Produkte von Caspasereaktionen binden können, können entweder einen Farbstoffanteil, ein kolloidales Metall (z.B. Silber, Gold), ein radioaktives Isotop und/oder Seltenerdenmetallchelate aufweisen.

Die kumulierte Caspaseaktivität wird insbesondere frühestens 10 h (insbesondere 24 bis 48 h) nach dem Versetzen der Probe mit der mindestens einen Substanz gemessen, da erst dann auch langsam wirkende Zytostatika Apoptose ausgelöst haben. Da Apoptose ein sehr früher Marker für die Wirksamkeit von Zytostatika ist, ist der beschriebene Test deutlich schneller als z.B. der MTT-Test mit 4 Tagen Inkubationsdauer.

Es ist wichtig, die Caspaseaktivität gleicher Zellzahlen nach Apoptose zu vergleichen. Es empfiehlt sich daher, das Ausmaß der Apoptose auf die Gesamtzahl der untersuchten Zellen zu normieren. Dies kann z.B. durch Messung der Lichtabsorption, Streulicht, Leitfähigkeitsmessung oder mikroskopisches Auszählen geschehen. Die Normierung ist notwendig, um das Ausmaß der Zellschädigung durch verschiedene Zytostatika vergleichen zu können.

Das erfindungsgemäße Verfahren ist insbesondere geeignet zur Stratifizierung von Tumorerkrankungen. Eine Klassifizierung der Tumorerkrankungen wird erfindungsgemäß ermöglicht, aus der sich ergibt, welches bestehende Standardprotokoll für eine Chemotherapie geeignet ist. Der erfindungsgemäße Test erlaubt es relativ schnell festzustellen, ob eine bestimmte Tumorzelllinie auf ein Chemotherapeutikum anspricht. In entsprechender Weise ist die Entwicklung von neuen Protokollen für die Chemotherapie von Tumorerkrankungen möglich, indem das erfindungsgemäße Verfahren zur kumulierten Messung der Caspase-Aktivität als Indikator für das Absterben von Tumorzellen verwendet wird. Schließlich erlaubt das erfindungsgemäße Verfahren auch, die Optimierung einer individuellen Chemotherapie gegen Tumorerkrankungen, In dem Patiententumorgewebe mit Chemotherapeutika behandelt werden und deren Ansprechen auf diese Chemotherapeutika durch Apoptose der Tumorzellen erfindungsgemäß bestimmt wird.

### Beispiel:

Ein stark wirkendes Zytostatikum, das das Wachstum der Zellen unmittelbar stoppt und alle Zellen in Apoptose überführt, kann aufgrund der geringen Zellzahl deutlich weniger Apoptose aufweisen, als im Falle eines schwächer wirkenden Zytostatikums, bei dem sich die Zellen zunächst noch vermehren können, um dann erst später zu einem geringen Teil in Apoptose zu gehen. Erst die Normierung berücksichtigt die Apoptose in Bezug auf die Gesamtzellzahl und bewertet das stärker wirkende Zytostatikum richtig.

Die Chemosensitivitätstestung von Zellen lässt sich besonders vorteilhaft mit einem Kit durchführen. Dieser Kit beinhaltet einen Probenträger mit mehreren Probenkammern, wobei eine Probenkammer mindestens eine Substanz enthält. Pro Kit ist außerdem eine standardisierte Lösung eines Reagenz zur Messung der Caspase-Aktivität enthalten. Das Caspasesubstrat ist insbesondere Aminocoumarin-DEVD In einem Lysepuffer. Es kann auch ein Caspase-Aktivitäts-Standard, enthalten sein. Bei dem Probenträger kann es sich z.B. um eine handelsübliche Mikrotiterplatte handeln. Die zu testenden Substanzen hängen von der Applikation ab und können vorgefertigt in den Kammern des Probenträgers entweder in Lösung oder eingetrocknet vorliegen. Die Substanzen können dabei entweder als Trockensubstanz, in Lösung oder in Gegenwart von Matrixsubstanzen wie z.B. Salzen, Puffer, Kohlenhydraten, Carbonsäuren, Pyrimidinen, anorganischen oder organischen Nanopartikeln bis 1 µm Durchmesser vorliegen.
- Fig. 1: zeigt die kumulierte und konventionell gemessene Caspaseaktivität von Jurkat-Zellen, die 4 h mit Actinomycin D induziert wurden.
- Fig. 2: zeigt die unterschiedlichen Zeitverläufe der Apoptose in Jurkat- und U937-Zellen nach Induktion durch Actinomycin D.
- Figur 3: zeigt die Chemosensitivität von U937-Zellen gegen verschiedene Zytostatika.
- Figur 4: zeigt die Chemosensitivität von HL60-Zellen gegen verschiedene Zytostatika.
- Figur 5: zeigt die Chemosensitivität von PBMNC gegenüber Daunorubicin.
- Figur 6: zeigt die Chemosensitivität von PBMNC gegenüber Ara-C.

### Beispiele

### Beispiel 1

Die Caspaseaktivitäten von 250000 Jurkat-Zellen in 100 µl Medium wurden nach verschiedenen Inkubationszeiten mit 1 µg/ml Actinomycin D konventionell und nach dem erfindungsgemäßen Verfahren der kumulierten Caspaseaktivität bestimmt. Dazu wurden 100 µl Jurkat-Zellen (2.5x10⁶/ml) in Gegenwart von Actinomycin D bei 5 % CO₂ und 37°C in RPMI-Medium mit 10 % FCS + Pen-Strep inkubiert. Nach der Inkubation wurden die Zellen nach konventioneller Methode zentrifugiert und mit PBS gewaschen. Das Pellet wurde in 200 µl Lysepuffer resuspendiert, 10 min auf Eis inkubiert und bei -20°C eingefroren. Die kumulierte Caspaseaktivität wurde bestimmt, indem 100 µl der mit Actinomycin D inkubierten Zellen direkt in Suspension mit 100 µl 2x Lysepuffer gemischt werden. Das Lysat wurde ebenfalls bei -20°C eingefroren. Alle Lysate wurden gemeinsam aufgetaut und mit Aminocoumarin-DEVD versetzt. Bei Raumtemperatur wurde die Freisetzung von fluoreszentem Aminocoumarin durch die Caspase alle 5 min. über eine Dauer von 2 h verfolgt. Aus dem linearen Signalanstieg wurde die Steigung als Maß für die Caspaseaktivität berechnet. Die in der Figur nicht gezeigten spontanen Caspase-Aktivitäten der Jurkat-Zellen in Abwesenheit von Actinomycin D nehmen mit der Zeit zu, bleiben aber kleiner als 4 min⁻¹.

### Beispiel 2

Die Caspaseaktivität von Jurkat- und U-937-Zellen wurde nach unterschiedlichen Inkubationszeiten mit Actinomycin D bestimmt. Dazu wurden je 0.5x10⁶ Zellen in 1 ml RPMI 1640 Medium mit 10 % FCS und Pen/Strep zwischen 30 Minuten und 16 Stunden mit 1 µg/ml Actinomycin D auf Zellkulturplatten (48 well) inkubiert. Anschließend wurden die Zellen in Eppendorf-Cups überführt und zweimal mit Medium gewaschen. Dann wurde das Pellet in Lysepuffer resuspendiert. Nach 10 Minuten auf Eis wurden 50 µM Aminocoumarin-DEVD-Substrat zugegeben und die Caspaseaktivität hier bei 37°C alle 5 min über einen Zeitraum von 2 Stunden verfolgt. Die Fluoreszenz wurde bei 355 nm angeregt und die Emission bei 460 nm gemessen. Aufgetragen ist der zeitliche Anstieg der Fluoreszenzemission gegen die Inkubationszeit mit Actinomycin D.

### Beispiel 3

### Chemosensitivität von Tumorzell-Linien

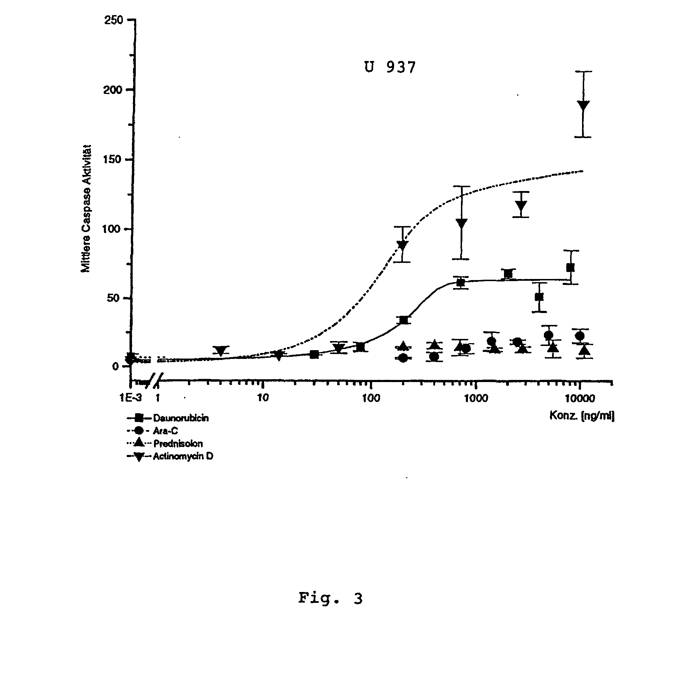

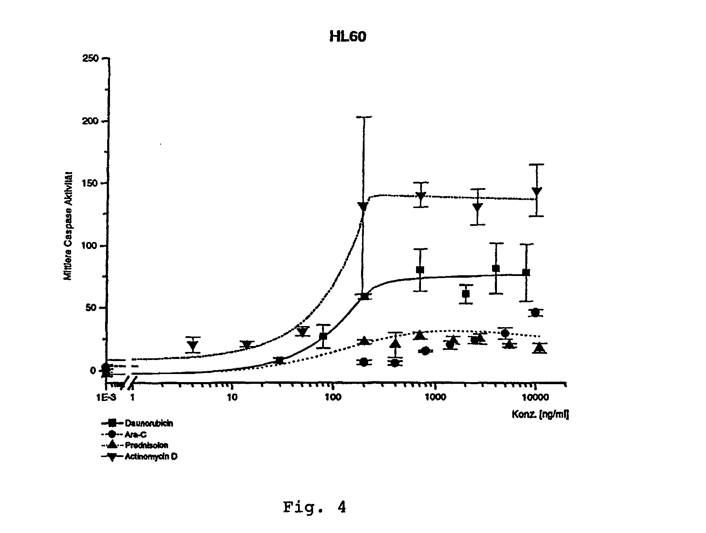

Die Chemosensitivitäten von U937- und HL60-Zellen gegen die in den Figuren 3 und 4 bezeichneten Zytostatika wurden in den angegebenen Konzentrationsbereichen getestet. Aus den Figuren wird deutlich, dass die beiden Tumorzell-Linien unterschiedlich stark auf Zytostatika ansprechen, wie es auch im Fall von primären Patientenzellen zu erwarten wäre.

Es wurde die Wirkung von 4 Zytostatika auf die Zell-Linie U937 getestet. Jeder Datenpunkt repräsentiert das Mittel aus drei unabhängigen Experimenten. Mit steigender Konzentration steigt die Wirksamkeit von Actinomycin D, Daunorubicin und Ara C, während Prednisolon keine oder nur eine geringe Apoptoseinduktion verursacht. Die maximal induzierbare Caspaseaktivität ist bei Ara-C relativ niedrig, während sie bei Daunorubicin deutlich höher und am größten bei Actinomycin D ist.

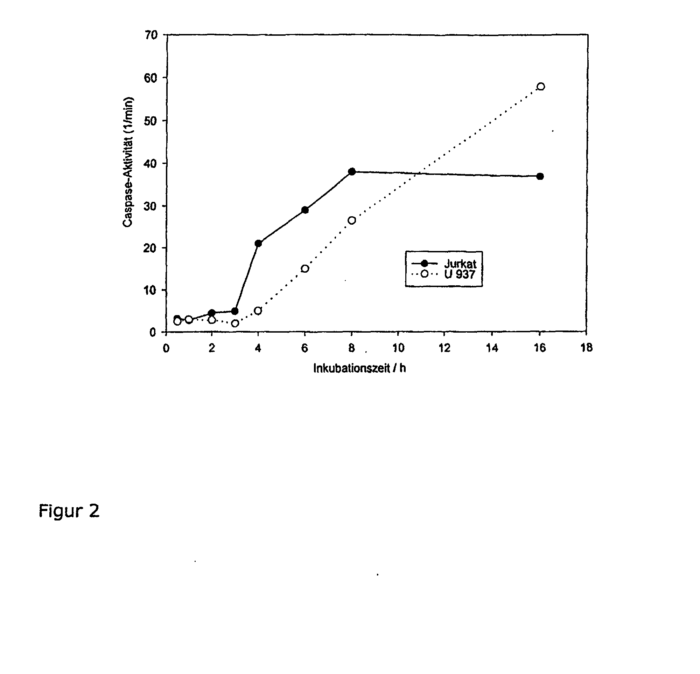

Zum Vergleich ist die Chemosensitivität von promyeloischen HL60-ZelIen in Abb. 2 gezeigt. Auch hier induzieren Actinomycin D und Daunorubicin größere Caspaseaktivitäten, als Ara-C und Prednisolon. Prednisolon zeigt ein Maximum in der Caspaseaktivität bei ca. 700 ng/ml. Bei höheren Konzentrationen sinkt das Apoptosesignal wieder ab. Ara-C scheint bei der höchsten gemessenen Konzentration noch nicht die maximal durch dieses Zytostatikum induzierbare Caspaseaktivität erreicht zu haben.

### Beispiel 4

### Chemosensitivität von PBMNC

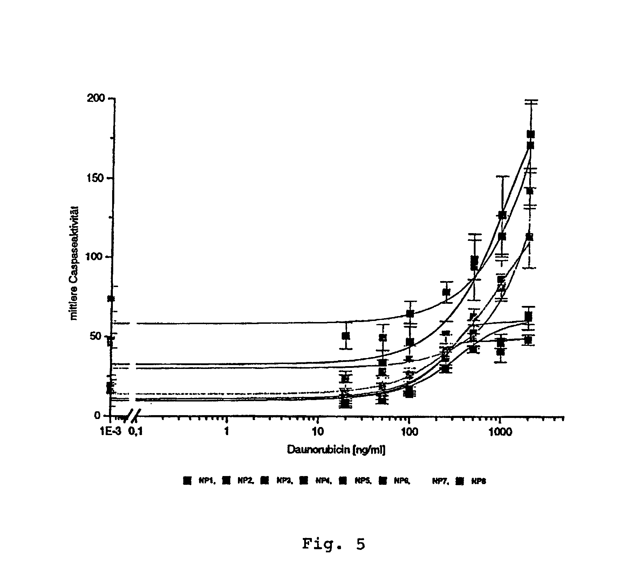

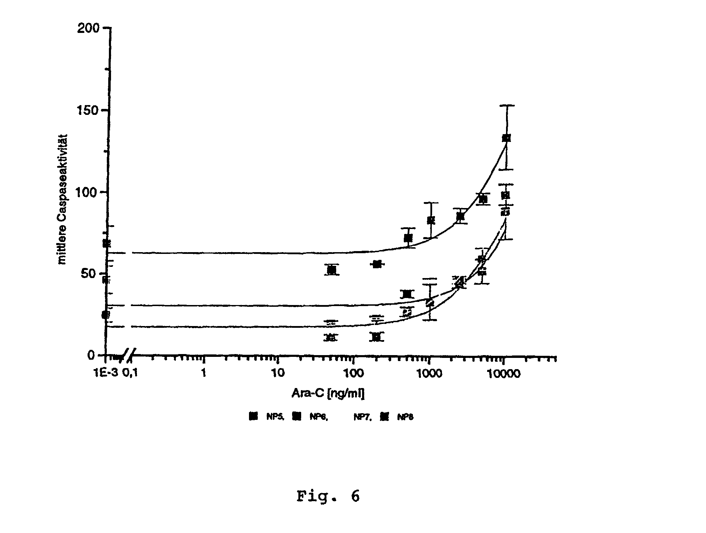

Zur Etablierung des Tests für peripheres Blut bzw. Knochenmark wurde zuerst isolierte Zellen des peripheren Blutes (PBMNC) von freiwilligen, gesunden Probanden getestet, wobei Wert darauf gelegt wurde, dass eine akute Infektion, wie sie in den Herbst-/Wintermonaten verstärkt auftritt, ausgeschlossen werden konnte. Die Figuren 5 und 6 stellen die Ergebnisse der Untersuchungen mit zwei Zytostatika dar.

In Figur 5 ist die Wirkung von Daunorubicin auf die mononukleären Zellen von acht Probanden (NP) zu sehen, wobei jeweils der Mittelwert ± Standardabweichung aus drei unabhängigen Experimenten gezeigt ist. Eine Caspaseaktivität ist durch >50 ng/ml Daunorubicin induzierbar, ein Plateau, d.h. eine auf dem Maximum gleichbleibende Caspaseaktivität wird jedoch bei den Personen NP1 und NP5 bis NP8 nicht und bei den Personen NP2 bis NP4 nur ansatzweise erreicht. Im Vergleich zu getesteten Leukämie-Zellinien, reagieren die PBMNC später auf Daunorubicin.

Figur 6 zeigt ist die Wirkung von Ara-C auf die mononukleären Zellen der Probanden NP5 bis NP8; dargestellt ist jeweils der Mittelwert ± Standardabweichung aus drei unabhängigen Experimenten. Eine Caspaseaktivität ist durch >200 ng/ml Ara-C induzierbar, ein Plateau, d.h. eine auf dem Maximum gleichbleibende Caspaseaktivität wird jedoch bei keinen der getesteten PBMNC erreicht.
Im Vergleich zu den Daten der Zelllinien HL60 und U937 (Beispiel 3) wird erkennbar, dass die maximal induzierbare Caspaseaktivität bei den zwei Zelllinien niedriger ist.

## Patentansprüche

1. Verfahren zur Bestimmung der Chemosensitivität von Zellen gegen mindestens eine Substanz durch Messung der durch die mindestens eine Substanz ausgelösten Apoptose, wobei die Apoptose durch kumulierte Caspase-Aktivität in einer Probe mit Zellen und einem Medium bestimmt wird, indem die Probe mit der mindestens einen Substanz versetzt und inkubiert wird und nach Zerstörung der Zellen ohne vorherige Abtrennung der Zellen die kumulierte Caspase-Aktivität in der Probe gemessen wird.

2. Verfahren gemäß Anspruch 1, wobei die Zellen tierische oder humane Zellen sind, insbesondere Leukämiezellen, Zellen solider Tumore, Zellen pathologischer Organe und/oder Referenzzellen wie beispielsweise Zellen aus anderen als den pathologischen Organen oder Zellen aus gesunden Bereichen pathologischer Organe.

3. Verfahren nach einem der Ansprüche 1 bis 2, **dadurch gekennzeichnet, dass** als Substanzen pharmazeutische Wirksubstanzen, Chemotherapeutika, Umweltschadstoffe, Peptide, Nukleinsäuren oder Derivate hiervon, PNAs und/oder Nukleinsäurehybride eingesetzt werden.

4. Verfahren nach einem der Ansprüche 1 bis 3, wobei die Caspase-Aktivität durch Substratumsatz von fluorogenen oder chromogenen Substraten oder durch Bindung von spezifischen Markern wie Antikörpern, F_{ab}-Fragmenten, single chain Antikörpern, Aptameren (Strukturen bindende Nukleinsäuren) und/oder sonstigen Proteinen mit Bindungsstellen für entweder unveränderte (Edukte) oder umgesetzte Caspase-Substrate (Produkte) gemessen wird.

5. Verfahren nach einem der Ansprüche 1 bis 4, **dadurch gekennzeichnet, dass** der Marker einen Farbstoffanteil, ein kolloidales Edelmetall, ein radioaktives Isotop und/oder Seltenerdenmetallchelate aufweist.

6. Verfahren nach einem der Ansprüche 1 bis 5, wobei die kumulierte Caspase-Aktivität frühestens 10 h, insbesondere 24 bis 48 h nach dem Versetzen der Probe mit der mindestens einen Substanz gemessen wird.

7. Verfahren nach einem der Ansprüche 1 bis 6, wobei die gemessene Caspase-Aktivität auf die Gesamtzellzahl normiert wird.

8. Verfahren nach einem der Ansprüche 1 bis 7, wobei die Messung der Caspase-Aktivität zur Stratifizierung von Tumorerkrankungen, zur Entwicklung von neuen Chemotherapien von Tumorerkrankungen und/oder Optimierung einer individuellen Chemotherapie gegen Tumorerkrankungen eingesetzt wird.

9. Kit zur Bestimmung der Chemosensitivität von Zellen, enthaltend einen Probenträger mit Probenkammern, wobei die Probenkammern mindestens eine zu testende Substanz enthalten sowie eine standardisierte Lösung eines Reagenz zur Messung der Caspase-Aktivität.

10. Kit nach Anspruch 9, wobei die Substanzen als Trockensubstanz, in Lösung oder in Gegenwart von Matrixsubstanzen vorliegen.

11. Kit nach einem der Ansprüche 9 und/oder 10, wobei die Matrixsubstanzen Salze, Puffer, Kohlenhydrate, Carbonsäuren, Pyrimidine, anorganische oder organische Nanopartikel bis 1 µm Durchmesser sein können.

## Claims

1. A method for determining the chemosensitivity of cells towards at least one substance by measuring the apoptosis induced by said at least one substance, wherein the apoptosis is determined from the accumulated caspase activity in a sample comprising cells and a medium by adding said at least one substance to the sample followed by incubation, and measuring the accumulated caspase activity in the sample upon disruption of the cells without previously separating off the cells.

2. The method according to claim 1, wherein said cells are animal, including human, cells, especially leukemia cells, cells of solid tumors, cells of pathological organs, and/or reference cells, such as cells from organs other than the pathological ones, or cells from healthy regions of pathological organs.

3. The method according to any of claims 1 to 2, **characterized in that** pharmaceutically active substances, chemotherapeutic agents, environmental pollutants, peptides, nucleic acids or derivatives thereof, PNAs and/or nucleic acid hybrides are employed as said substances.

4. The method according to any of claims 1 to 3, wherein the caspase activity is measured through the substrate turnover rate of fluorogenic or chromogenic substrates, or through the binding of specific markers, such as antibodies, F_{ab} fragments, single-chain antibodies, aptamers (structure-binding nucleic acids), and/or other proteins having binding sites for either unchanged (educts) or converted (products) caspase substrates.

5. The method according to any of claims 1 to 4, **characterized in that** said marker comprises a dye portion, a colloidal precious metal, a radioactive isotope, and/or rare-earth metal chelates.

6. The method according to any of claims 1 to 5, wherein the accumulated caspase activity is measured no sooner than 10 h, especially from 24 to 48 h, after said at least one substance has been added to the sample.

7. The method according to any of claims 1 to 6, wherein the measured caspase activity is standardized for the total number of the cells.

8. The method according to any of claims 1 to 7, wherein the measurement of caspase activity is employed for the stratification of tumor diseases, for developing new chemotherapies of tumor diseases, and/or for the optimization of an individual chemotherapy against tumor diseases.

9. A kit for determining the chemosensitivity of cells, containing a sample support with sample compartments, each of the sample compartments containing at least one substance to be tested, and a standardized solution of a reagent for measuring caspase activity.

10. The kit according to claim 9, said substances being present as dry substances, in solution, or in the presence of matrix substances.

11. The kit according to any of claims 9 and/or 10, wherein said matrix substances are selected from salts, buffers, carbohydrates, carboxylic acids, pyrimidines, inorganic or organic nanoparticles with diameters of up to 1 µm.

## Revendications

1. Procédé de détermination de la chimlosensibilité de cellules vis à vis d'au moins une substance par mesure de l'apoptose déclenchée par la au moins une substance, dans lequel on détermine l'apoptose par l'activité de la caspase cumulée dans un échantillon comportant des cellules et un milieu, l'échantillon étant ajouté avec la au moins une substance et mis à incuber, et l'activité de la caspase cumulée étant mesurée dans l'échantillon, après destruction des cellules. sans effectuer de séparation préalable des cellules.

2. Procédé selon la revendication 1, dans lequel les cellules sont des cellules animales ou humaines, notamment des cellules leucémiques, des cellules de tumeurs solides, des cellules d'organes pathologiques et/ou des cellules de référence comme par exemple des cellules provenant d'autres organes non pathologiques ou des cellules provenant de régions saines d'organes pathologiques.

3. Procédé selon une des revendications 1 à 2, **caractérisé en ce qu'**on utilise en tant que substances des substances actives pharmaceutiques, des agents chimiothérapiques, des substances polluantes pour l'environnement, des peptides, des acides nucléiques ou des dérivés d'entre eux, des ANP et/ou des hybrides d'acide nucléique.

4. Procédé selon une des revendications de 1 à 3, **caractérisé en ce qu'**on mesure l'activité de la caspase par la transformation du substrat de substrats fluorogènes ou chromogènes ou par liaison de marqueurs spécifiques tels que des anticorps, des fragments F_{ab}, des anticorps à une seule chaîne, des aptamères (Acides nucléiques se liant à des structures) et/ou des protéines quelconques comportant des sites de liaison à des substrats de caspase non modifiés (éduits) ou transformés (produits).

5. Procédé selon une des revendications de 1 à 4, **caractérisé en ce que** le marqueur comporte une portion colorante, un métal noble colloïdal, un isotope radioactif, et/ou un chélate de métal de terre rare.

6. Procédé selon une des revendications de 1 à 5, dans lequel on mesure l'activité de la caspase cumulée au plus tôt 10 h, en particulier de 24 à 48 h, après un ajout de la au moins une substance dans l'échantillon.

7. Procédé selon une des revendications de 1 à 6, dans lequel l'activité de la caspase mesurée est nonnée sur le nombre total de cellules.

8. Procédé selon une des revendications de 1 à 7, dans lequel on utilise la mesure de l'activité de la caspase pour stratifier des maladies tumorales, pour développer de nouvelles chimiothérapies pour des maladies tumorales et /ou pour optimiser une chimiothérapie individuelle contre des maladies tumorales.

9. Kit de détermination de la chimiosensibilité des cellules, comportant un support d'échantillon doté de compartiments à échantillon, dans lequel les compartiments à échantillon contiennent au moins une substance à tester ainsi qu'une solution standardisée d'un réactif destiné à la mesure de l'activité de la caspase.

10. Kit selon la revendication 9, dans lequel les substances sont sous forme de substance sèche, de solution ou sont contenues dans une substance matrice.

11. Kit selon une des revendications 9 et/ou 10, dans lequel les substances matrices peuvent être des sels, des tampons, des carbohydrates, des acides carboxyliques, des pyrimidines, ou des nanoparticules minérales ou organiques d'un diamètre maximal de 1 µm.
